# EUROPEAN PATENT SPECIFICATION

(11) **EP 3 560 910 B1**
(45) Date of publication and mention of the grant of the patent: **29.03.2023**
(21) Application number: 17886363.5
(22) Date of filing: 22.12.2017
(51) Int. Cl.: C07C 67/30, C07C 69/62, C07C 17/18, C07C 22/08, C07C 19/08, C07C 23/10, C07C 17/093, C07C 67/307

(54) **METHOD FOR MANUFACTURING DIFLUOROMETHYLENE COMPOUND**
VERFAHREN ZUR HERSTELLUNG EINER DIFLUORMETHYLENVERBINDUNG
PROCÉDÉ DE FABRICATION DE COMPOSÉ DIFLUOROMÉTHYLÈNE

(30) Priority: 26.12.2016 JP 2016251315; 13.11.2017 JP 2017218128
(43) Date of publication of application: 30.10.2019
(73) Proprietor: DAIKIN INDUSTRIES, LTD., Osaka-shi, Osaka 530-0001 (JP)
(72) Inventor: OHTSUKA, Tatsuya, Osaka-shi Osaka 530-0001 (JP); KUROKI, Yoshichika, Osaka-shi Osaka 530-0001 (JP); SHIRAI, Atsushi, Osaka-shi Osaka 530-0001 (JP); HOSOKAWA, Moe, Osaka-shi Osaka 530-0001 (JP); KISHIKAWA, Yosuke, Osaka-shi Osaka 530-0001 (JP)
(74) Representative: Hoffmann Eitle
(86) International application number: PCT/JP2017/046195
(87) International publication number: WO 2018/123890

(56) References cited:
- EP-A1- 1 304 316
- WO-A1-01/96263
- WO-A2-2008/014345
- SHOJI HARA ET AL: "IF5-pyridine-HF: air- and moisture-stable fluorination reagent", TETRAHEDRON, vol. 68, no. 49, 1 January 2012 (2012-01-01), pages 10145-10150, XP55612631, AMSTERDAM, NL ISSN: 0040-4020, DOI: 10.1016/j.tet.2012.09.104
- HARA, SHOJI et al.: "IF5 pyridine HF: air- and moisture-stable fluorination reagent", Tetrahedron, vol. 68, no. 49, 2012, pages 10145-10150, XP055612631, ISSN: 0040-4020, DOI: 10.1016/j.tet.2012.09.104
- TULLOCK, C. W. et al.: "The Chemistry of Sulfur Tetrafluoride I . The Synthesis of Sulfur Tetrafluoride", J. Am. Chem. Soc., vol. 82, no. 3, 5 February 1960 (1960-02-05), pages 539-542, XP055612656,
- STEVENS, T. E.: "Fluorination of Some Nitriles and Ketones with Bromine Trifluoride", J. Org. Chem., vol. 26, no. 5, 1961, pages 1627-1630, XP055612660,
- HASEK, W. R. et al.: "The Chemistry of Sulfur Tetrafluoride. II. The Fluorination of Organic Carbonyl Compounds", J. Am. Chem. Soc., vol. 82, no. 3, 5 February 1960 (1960-02-05), pages 543-551, XP002125300, ISSN: 0002-7863, DOI: 10.1021/ja01488a012
- MIDDLETON, W. J. et al.: "New fluorinating reagents. Dialkylaminosulfur fluorides", J. Org. Chem., vol. 40, no. 5, 1975, pages 574-578, XP002125274, ISSN: 0022-3263, DOI: 10.1021/jo00893a007
- LAL, G. S. et al.: "Bis(2-methoxyethyl)aminosulfur Trifluoride: A New Broad-Spectrum Deoxofluorinating Agent with Enhanced Thermal Stability", J. Org. Chem., vol. 64, no. 19, 1999, pages 7048-7054, XP003003212, ISSN: 0022-3263

## Description

### Technical Field

The present invention relates to a method for producing a difluoromethylene compound.

### Background Art

Compounds having a difluoromethylene skeleton (i.e., difluoromethylene compounds) are useful as e.g. a liquid crystal material, a medicinal drug, and intermediates thereof. Although studies have been made on various production methods, deoxyfluorination of carbonyl compound, in particular, is a useful reaction. Fluorinating agents known to be suitable for such a reaction include sulfur tetrafluoride (SF4), N,N-diethylaminosulfur trifluoride (DAST), bis(methoxymethyl)aminosulfur trifluoride (Deoxo-Fluor, trade name) and substituted phenylsulfur trifluoride (Fluolead, trade name).

However, SF4 is highly toxic and is in the form of gas; thus, it is difficult to handle and obtain. DAST and Deoxo-fluor are liquids that have low thermal stability and generate a very large amount of thermal energy when decomposed. In particular, DAST is explosive and requires caution when handled. Although Fluolead is highly stable, there is a problem in that sulfur compounds produced as a byproduct by decomposition of a fluorinating agent are not easily separated from the reaction product.

WO 2008/014345 discloses a process of converting a carbonyl group into a -CF₂ moiety using phenyl sulfur trifluorides as fluorination agents.

### Citation List

PTL 1: US 7,265,247
NPL 1: J. Am. Chem. Soc., 82, and 543 (1960)
NPL 2: J. Org. Chem., 40, and 574 (1975)
NPL 3: Chemical Communications 215 (1999)

### Summary of Invention

### Technical Problem

An object of the present invention is to provide a novel method for producing a difluoromethylene compound, in particular, a simple method for producing a difluoromethylene compound.

### Solution to Problem

As a result of extensive research, the present inventors found that the above problem can be solved by a production method comprising step A of mixing:
a) a carbonyl compound containing a -C(O)- moiety;
b) optionally an amine;
c) a fluoride of the formula MF, wherein M is a Group 1 element of the periodic table;
d) IF₅; and
e) sulfur chloride.

The present invention has been accomplished based on this finding. Preferred embodiments of the invention are as defined in the appended dependent claims and/or in the following detailed description.

### Advantageous Effects of Invention

The present invention provides a novel method for producing a difluoromethylene compound, in particular, a simple, efficient method for producing a difluoromethylene compound.

### Description of Embodiments

### Term

The symbols and the abbreviations in this specification are to be interpreted as having the general meanings in the related technical field to which the present invention pertains, according to the context of this specification, unless otherwise specified. Also, in the present specification the following definitions apply.

The term "comprise" or "contain" is intended to encompass the meanings of "consist essentially of" and "consist of."

The steps, treatments, or operations in this specification can be performed at room temperature, unless otherwise specified; and room temperature refers to a temperature of 10-40°C.

The term "Cₙ-ₘ" (wherein n and m are numbers) indicates that the carbon number is n or more and m or less, as usually would be understood by a person skilled in the art.

Examples of "non-aromatic hydrocarbon ring" include non-aromatic C₃₋₈-hydrocarbon rings.
Specific examples include:
(1) C₃₋₈-cycloalkanes, such as cyclopropane, cyclobutane, cyclopentane, cyclohexane, cycloheptane, and cyclooctane;
(2) C₅-C₈ cycloalkenes, such as cyclopentene, cyclohexene, cycloheptene, and cyclooctene;
(3) C₅₋₈-cycloalkadienes, such as cyclopentadiene, cyclohexadiene, cycloheptadiene, and cyclooctadiene;
(4) bridged-ring C₅₋₈-hydrocarbons, such as bicyclo[2.1.0]pentane, bicyclo[2.2.1]heptane, bicyclo[3.2.1]octane, bicyclo[2.2.1]hept-2-ene, and tricyclo[2.2.1.0]heptane.

Examples of "non-aromatic heterocycle" include 3- to 8-membered non-aromatic heterocycles. Specific examples include oxirane, azetidine, oxetane, thietane, pyrrolidine, dihydrofuran, tetrahydrofuran, tetrahydrothiophene, imidazolidine, oxazolidine, isoxazoline, piperidine, dihydropyran, tetrahydropyran, tetrahydrothiopyran, morpholine, thiomorpholine, piperazine, dihydrooxazine, tetrahydrooxazine, dihydropyrimidine, tetrahydropyrimidine, azepane, oxepane, thiepane, oxazepane, thiazepane, azocane, oxocane, thiocane, oxazocane and thiazocane.

The term "organic group" refers to a group containing at least one carbon atom, or a group formed by removing one hydrogen atom from an organic compound.

Examples of the "organic group" include cyano, aldehyde, R, RO-, RCO-, RSO₂-, ROCO- and ROSO₂-, wherein R aeach independently is hydrocarbon, non-aromatic heterocyclic group or heteroaryl, each optionally having at least one substituent.

Examples of "hydrocarbon" include alkyl, alkenyl, alkynyl, cycloalkyl, cycloalkenyl, cycloalkadienyl, aryl, aralkyl, and groups of combinations thereof.

"(Cyclo)alkyl" refers to alkyl and/or cycloalkyl.

Examples of "alkyl" include linear or branched C₁₋₁₀-alkyl, such as methyl, ethyl, propyl, isopropyl, butyl, isobutyl, sec-butyl, tert-butyl, pentyl, isopentyl, neopentyl, hexyl, heptyl, octyl, nonyl and decyl.

Examples of "alkenyl" include linear or branched C₂₋₁₀-alkenyl, such as vinyl, 1-propen-1-yl, 2-propen-1-yl, isopropenyl, 2-buten-1-yl, 4-penten-1-yl and 5-hexen-1-yl.

Examples of "alkynyl" include linear or branched C₂-C₁₀ alkynyl, such as ethynyl, 1-propyn-1-yl, 2-propyn-1-yl, 4-pentyn-1-yl, and 5-hexyne-1-yl.

Examples of "cycloalkyl" include C₃-C₇ cycloalkyl, such as cyclopropyl, cyclobutyl, cyclopentyl, cyclohexyl and cycloheptyl.

Examples of "cycloalkenyl" include C₃-C₇ cycloalkenyl, such as cyclopropenyl, cyclobutenyl, cyclopentenyl, cyclohexenyl and cycloheptenyl.

Examples of "cycloalkadienyl" include C₄-C₁₀ cycloalkadienyl, such as cyclobutadienyl, cyclopentadienyl, cyclohexadienyl, cycloheptadienyl, cyclooctadienyl, cyclononadienyl and cyclodecadienyl.

"Aryl" may be monocyclic, bicyclic, tricyclic, or tetracyclic, unless otherwise specified.

"Aryl" may be C₆₋₁₈-aryl, unless otherwise specified.

Examples of "aryl" include phenyl, 1- and 2-naphthyl, 2-, 3- and 4-biphenyl and 2-anthryl.

Examples of "aralkyl" include benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, 2-biphenylylmethyl, 3-biphenylylmethyl and 4-biphenylylmethyl.

"Non-aromatic heterocyclic group" may be mono-, bi-, tri- or tetracyclic, unless otherwise specified.

"Non-aromatic heterocyclic group" may be, for example, a non-aromatic heterocyclic group containing, in addition to carbon, 1-4 heteroatoms selected from oxygen, sulfur, and nitrogen as a ring-constituting atom or ring-constituting atoms.

"Non-aromatic heterocyclic group" may be saturated or unsaturated.

Examples of "non-aromatic heterocyclic group" include tetrahydrofuryl, oxazolidinyl, imidazolinyl (e.g., 1-, 2- and 4-imidazolinyl), aziridinyl (e.g., 1- and 2-aziridinyl), azetidinyl (e.g., 1- and 2-azetidinyl), pyrrolidinyl (e.g., 1-, 2- and 3-pyrrolidinyl), piperidinyl (e.g., 1-, 2- and 3-piperidinyl), azepanyl (e.g., 1-, 2-, 3- and 4-azepanyl), azocanyl (e.g., 1-, 2-, 3- and 4-azocanyl), piperazinyl (e.g., 1,4-piperazin-1-yl and -2-yl), diazepinyl (e.g., 1,4-diazepin-1-yl, -2-yl, -5-yl and -6-yl), diazocanyl (e.g., 1,4-diazocan-1-yl, -2-yl -5-yl and -6-yl, and 1,5-diazocan-1-yl, -2-yl and -3-yl), tetrahydropyranyl (e.g., tetrahydropyran-4-yl), morpholinyl (e.g., 4-morpholinyl), thiomorpholinyl (e.g., 4-thiomorpholinyl), 2-oxazolidinyl, dihydrofuryl, dihydropyranyl and dihydroquinolyl.

Examples of "heteroaryl" include 5- or 6-membered monocyclic aromatic heterocyclic groups and 5- to 10-membered aromatic fused heterocyclic groups.

Examples of "5- or 6-membered monocyclic aromatic heterocyclic group" include pyrrolyl (e.g., 1-, 2- and 3-pyrrolyl), furyl (e.g., 2- and 3-furyl), thienyl (e.g., 2- and 3-thienyl), pyrazolyl (e.g., 1-, 3- and 4-pyrazolyl), imidazolyl (e.g., 1-, 2- and 4-imidazolyl), isoxazolyl (e.g., 3-, 4- and 5-isoxazolyl), oxazolyl (e.g., 2-, 4- and 5-oxazolyl), isothiazolyl (e.g., 3-, 4- and 5-isothiazolyl), thiazolyl (e.g., 2-, 4- and 5-thiazolyl), triazolyl (e.g., 1,2,3-triazol-4-yl and 1,2,4-triazol-3-yl), oxadiazolyl (e.g., 1,2,4-oxadiazol-3-yl and -5-yl), thiadiazolyl (e.g., 1,2,4-thiadiazol-3-yl and -5-yl), tetrazolyl, pyridyl (e.g., 2-, 3- and 4-pyridyl), pyridazinyl (e.g., 3- and 4-pyridazinyl), pyrimidinyl (e.g., 2-, 4- and 5-pyrimidinyl) and pyrazinyl.

Examples of "5- to 10-membered aromatic fused heterocyclic group" include isoindolyl (e.g., 1-, 2-, 3-, 4-, 5-, 6- and 7-isoindolyl), indolyl (e.g., 1-, 2-, 3-idolyl, 4-, 5-, 6-and 7-indolyl), benzo[b]furanyl (e.g., 2-, 3-, 4-, 5-, 6- and 7-benzo[b]furanyl), benzo[c]furanyl (e.g., 1-, 4- and 5-benzo[c]furanyl), benzo[b]thienyl (e.g., 2-, 3-, 4-, 5-, 6- and 7-benzo[b]thienyl), benzo[c]thienyl (e.g., 1-, 4- and 5-benzo[c]thienyl), indazolyl (e.g., 1-, 2-, 3-, 4-, 5-, 6- and 7-indazolyl), benzimidazolyl (e.g., 1-, 2-, 4- and 5-benzimidazolyl), 1,2-benzisoxazolyl (e.g., 1,2-benzisoxazol-3-yl, -4-yl, -5-yl, -6-yl and -7-yl), benzoxazolyl (e.g., 2-, 4-, 5-, 6- and 7-benzoxazolyl), 1,2-benzisothiazolyl (e.g., 1,2-benzisothiazol-3-yl, -4-yl, -5-yl, -6-yl and -7-yl), benzothiazolyl (e.g., 2-, 4-, 5-, 6- and 7-benzothiazolyl), isoquinolyl (e.g., 1-, 3-, 4- and 5-isoquinolyl), quinolyl (e.g., 2-, 3-, 4-, 5- and 8-quinolyl), cinnolinyl (e.g., 3-, 4-, 5-, 6-, 7- and 8-cinnolinyl), phthalazinyl (e.g., 1-, 4-, 5-, 6-, 7- and 8-phthalazinyl), quinazolinyl (e.g., 2-, 4-, 5-, 6-, 7- and 8-quinazolinyl), quinoxalinyl (e.g., 2-, 3-, 5-, 6-, 7- and 8-quinoxalinyl), pyrazolo[1,5-a]pyridyl (e.g., pyrazolo[1,5-a]pyridin-2-yl, -3-yl, -4-yl, -5-yl, -6-yl and -7-yl), and imidazo[1,2-a]pyridyl (e.g., imidazo[1,2-a]pyridin-2-yl, -3-yl, - 5-yl, -6-yl, -7-yl and -8-yl.

Examples of "halogen atom" include F, Cl, Br and I.

Examples of the term "halogenated" can include the meanings of chlorinated, brominated, and iodinated.

### Production Method

The method for producing a difluoromethylene compound containing a -CF₂- moiety (sometimes simply referred to as "the difluoromethylene compound") of the present invention comprises step A of mixing:
a) a carbonyl compound containing a -C(O)- moiety (sometimes referred to as "the carbonyl compound a)");
b) optionally an amine;
c) a fluoride of the formula MF, wherein M is a Group 1 element of the periodic table (sometimes referred to as "the fluoride c) ") ;
d) IF₅; and
e) sulfur chloride.

The term "difluoromethylene" in the difluoromethylene compound produced by the present method refers to the -CF₂- moiety.

The difluoromethylene compound can contain one or more -CF₂- moieties, and corresponding to this, the carbonyl compound a) can contain one or more -C(O)- moieties.
a) The carbonyl compound used in the present method is converted in step A into a difluoromethylene compound containing a -CF₂- moiety.

It is preferable that a compound in which an organic group is bonded to the -C(O)- moiety via -O- (i.e., an ester compound) is excluded from the carbonyl compound a), which is a reaction starting compound.

Accordingly, it is preferable that a compound in which an organic group is bonded to the -CF₂- moiety via -O- is excluded from the difluoromethylene compound, i.e., the reaction product.

The -CF₂- moiety in the difluoromethylene compound, i.e., the reaction product, may be, for example, part of a -CF₃ group. That is, the difluoromethylene compound may be a trifluoromethyl compound.

In regard to this, when -COOH is attached to the -C(O)-moiety in the carbonyl compound a), the -C(O)-COOH moiety can be converted into a -CF₃ group in step A. Therefore, in this case, a trifluoromethyl compound is produced by the present method.

The difluoromethylene compound produced by the present method is preferably represented by the formula R¹¹-CF₂-R¹² (1), Wherein R¹¹ is R²¹ or F, R² is R²² or F, R²¹ and R²² each independently are H or an organic group, or R²¹ and R²² taken together with the -CF₂- moiety to which they are attached, may form a ring; with the proviso that neither R¹¹ nor R¹², and neither R²¹ nor R²², is an organic group bonded via -O-.

Corresponding to this preferable difluoromethylene compound, the carbonyl compound a) is preferably a carbonyl compound of the formula R²¹-C(O)-R²² (2), wherein the symbols in the formula are as defined above.

R¹¹ in the target compound of formula (1) corresponds to R²¹ in the reaction starting compound of formula (2) , and R¹¹ and R²¹ may be the same.

R¹² in the target compound of formula (1) corresponds to R²² in the reaction starting compound of formula (2) a, and R¹² and R²² may be the same.

However, as is understood from the description above, in the method of this embodiment, when R²¹ in formula (2) above is -COOH, R¹¹ in formula (1) may be F.

Similarly, in the method of this embodiment, when R²² in formula (2) above is -COOH, R¹² in formula (1) may be F.

The organic group represented by R¹¹ is preferably hydrocarbon optionally having at least one substituent. (This hydrocarbon may contain at least one moiety selected from -NR-, =N-, -N=, -O-, and -S-, wherein R is H or an organic group.)

The "hydrocarbon" in the "hydrocarbon optionally having at least one substituent" is preferably C₁₋₃₀-hydrocarbon, more preferably C₁₋₂₀-hydrocarbon, and still more preferably C₁₋₁₀-hydrocarbon.

The hydrocarbon is preferably alkyl or aryl, and more preferably C₁₋₁₀-alkyl or C₆₋₂₀-aryl.

The hydrocarbon (including alkyl and aryl) may contain at least one moiety selected from -NR- (wherein R is H or an organic group), =N-, -N=, -O-, and -S-.

This moiety may be inserted in the carbon-carbon bonding of the hydrocarbon and/or inserted adjacent to the -CF₂-moiety in formula (1) (and corresponding to this, adjacent to the -C(O)- moiety in formula (2)).

The organic group represented by R in the -NR- moiety is preferably hydrocarbon optionally having at least one substituent, and the hydrocarbon in the "hydrocarbon optionally having at least one substituent" is preferably C₁₋₃₀-hydrocarbon, more preferably C₁₋₂₀-hydrocarbon, and still more preferably C₁₋₁₀-hydrocarbon.

The organic group represented by R¹² is preferably hydrocarbon optionally having at least one substituent. (This hydrocarbon may contain at least one moiety selected from -NR-, =N-, -N=, -O-, and -S-, wherein R is H or an organic group.)

The "hydrocarbon" in the "hydrocarbon optionally having at least one substituent" is preferably C₁₋₃₀-hydrocarbon, more preferably C₁₋₂₀-hydrocarbon, and still more preferably C₁₋₁₀-hydrocarbon.

The hydrocarbon is preferably alkyl or aryl, and more preferably C₁₋₁₀-alkyl or C₆₋₂₀-aryl.

The hydrocarbon (including alkyl and aryl) may contain at least one moiety selected from -NR- (wherein R is H or an organic group), =N-, -N=, -O-, and -S-.

This moiety may be inserted in the carbon-carbon bonding of the hydrocarbon and/or inserted adjacent to the -CF₂-moiety in formula (1).

The organic group represented by R in R¹² is preferably hydrocarbon optionally having at least one substituent, and the hydrocarbon in the "hydrocarbon optionally having at least one substituent" is preferably C₁₋₂₀-hydrocarbon, more preferably C₁₋₁₀-hydrocarbon, and still more preferably C₁₋₅-hydrocarbon.

As a person skilled in the art would usually understand, aryl containing at least one moiety selected from -NR-, =N-, -N=, -O-, and -S- may be heteroaryl.

Examples of the "substituent" in the "hydrocarbon optionally having at least one substituent" represented by R¹¹ or R²¹ include halogen atoms, nitro, cyano, oxo, thioxo, sulfo, sulfamoyl, sulfinamoyl, and sulfenamoyl.

The numbers of the substituents in R¹¹ and R²¹ may be the same or different and within a range of one to the maximum replaceable number (e.g., one, two, three, four, five, or six).

It is preferable that the ring formed by R¹¹ and R²¹, taken together with the -CF₂- moiety to which R¹¹ and R²¹ are attached, be a 3- to 8-membered ring optionally further having at least one substituent, in addition to fluorine in the -CF₂- moiety.

The ring may be a monocyclic, fused, or spiro ring.

The ring may be a non-aromatic hydrocarbon ring or non-aromatic heterocycle.

Examples of the substituent include halogen atoms, nitro, cyano, oxo, thioxo, sulfo, sulfamoyl, sulfinamoyl, and sulfenamoyl.

The number of the substituents may be within a range of one to the maximum replaceable number (e.g., one, two, three, four, five, or six).

The "organic group bonded via -O-" excluded from R²¹ and R²² is, for example, hydrocarbyloxy optionally having at least one substituent.

Examples of b) the amine include aliphatic amines (primary, secondary and tertiary amine), alicyclic amines (secondary and tertiary amine), aromatic amines (primary, secondary and tertiary amine), and heterocyclic amines; polyaryl amine, and polyvinylpyridine.

Examples of aliphatic primary amines include methylamine, ethylamine, propylamine, butylamine, pentylamine, hexylamine, cyclohexylamine and ethylenediamine.

Examples of aliphatic secondary amines include dimethylamine, diethylamine, dipropylamine, dibutylamine, dipentylamine, dihexylamine and dicyclohexylamine.

Examples of aliphatic tertiary amines include trimethylamine, triethylamine, diisopropylethylamine and N,N,N',N'-tetramethylethylenediamine.

Examples of alicyclic secondary amines include piperidine, piperazine, pyrrolidine and morpholine.

Examples of alicyclic tertiary amines include N-methylpiperazine, N-methylpyrrolidine, 5-diazabicycio[4,3,0]nonan-5-ene and 1,4-diazabicyclo[2,2,2]octane.

Examples of aromatic amines include aniline, methylaniline, dimethylaniline, N,N-dimethylaniline, haloaniline and nitroaniline.

Examples of heterocyclic amines include pyridine, pyrimidine, piperazine, quinoline and imidazole.

For the fluoride c), preferable examples of the Group 1 element of the periodic table represented by M in the formula MF include H, Na, and K, with H being particularly preferable. That is, a preferable example of the fluoride c) is HF.

The Group 1 element of the periodic table represented by M may be a cation.

The halogenated fluorine compound d) is IF₅.

Preferable examples of the sulfur chloride e)include sulfur monochloride (SCl or S₂Cl₂), and sulfur dichloride (SCl₂).

Preferable examples of the sulfur chloride include sulfur monochloride.

These may be used singly or in a combination of two or more.

In a preferable embodiment of the present invention,
a) the carbonyl compound containing a -C(O)- moiety,
d) IF₅, and
e) the sulfur chloride, as well as
b) an amine and
c) a fluoride represented by the formula: MF, wherein M represents a Group 1 element of the periodic table, are mixed.

The upper limit of the amount of the amine b)used in the reaction of step A is preferably 2 equivalents, more preferably 1.5 equivalents, and still more preferably 1 equivalent in a molar ratio relative to IF₅.

The lower limit of the amount of the amine b)used in the reaction of step A is preferably 0.5 equivalents, more preferably 0.8 equivalents, still more preferably 0.9 equivalents, and still even more preferably 1 equivalent in a molar ratio relative to IF₅.

The amount of the amine b) used in the reaction of step A is preferably 0.5-2 equivalents, more preferably 0.8-1.2 equivalents, and still more preferably 0.9-1.1 equivalents in a molar ratio relative to IF₅.

The upper limit of the amount of the fluoride c)used in the reaction of step A is preferably 9 equivalents, more preferably 7 equivalents, still more preferably 5 equivalents, and still even more preferably 3 equivalents in a molar ratio relative to the amine.

The lower limit of the amount of the fluoride c) used in the reaction of step A is preferably 2 equivalents, more preferably 2.5 equivalents, and still more preferably 3 equivalents in a molar ratio relative to the amine.

The amount of the fluoride c) used in the reaction of step A is preferably 2.5-9 equivalents, more preferably 2.5-7 equivalents, still more preferably 2.5-5 equivalents, and still even more preferably 3-5 equivalents in a molar ratio relative to the amine.

The upper limit of the amount of IF₅d) used in the reaction of step A is preferably 3 equivalents, more preferably 2.2 equivalents, and still more preferably 1.8 equivalents in a molar ratio relative to e) the sulfur chloride.

The lower limit of the amount of IF₅ used in the reaction of step A is preferably 1 equivalent, and more preferably 1.5 equivalents in a molar ratio relative to the sulfur chloride.

The amount of IF₅used in the reaction of step A is preferably 1-3 equivalents, more preferably 1.5-2.2 equivalents, and still more preferably 1.5-1.8 equivalents in a molar ratio relative to the sulfur chloride.

In step A, other substances may also be mixed in addition to the carbonyl compound a), the amine b), the fluoride c), as well as IF₅ and e) the sulfur chloride, which are optionally used.

Examples of the other substances include reaction solvents.

Specific examples of the reaction solvents include dichloromethane, tetrachloroethane, chloroform, carbon tetrachloride, cyclohexane, and mixed solvents of two or more of these.

The amount of such other substances may be suitably determined according to the purpose of use.

In the production method of the present invention, the carbonyl compound a), the amine b), and the fluoride c), as well as IF₅ d)and the sulfur chloride e), which are optionally added, are introduced or added simultaneously or sequentially to the reaction system of step A.

Therefore, in mixing in step A, the carbonyl compound a), the amine b), and the fluoride c), as well as IF₅and the sulfur chloride e), which are optionally added, are not necessarily present all together to be mixed.

More specifically, for example, 1 to 4 substances from among the 5 substances a) to e) may have undergone modification by e.g. chemical reaction before the substance(s) other than the 1 to 4 substances are added to the reaction system of step A.

In step A, all or part of one or more substances a) to e) may be used in the form of a precursor thereof, a salt thereof, or a complex thereof.

In a preferable embodiment of the present invention, the amine b) and the fluoride c) are used in the form of a salt thereof (a salt of an amine and hydrogen fluoride).

This salt can be used as all or part of the amine b) and the fluoride c).

In other words, at least one member selected from the amine b) and the fluoride c)can also be used, in addition to this salt.

Specific examples of the salt (a salt of an amine and HF) include salts of a primary secondary or tertiary amine and HF. In preferable examples the amine is an aliphatic amine.

Examples of aliphatic primary amines include methylamine, ethylamine, propylamine, butylamine, pentylamine and hexylamine.

Examples of aliphatic secondary amines include dimethylamine, diethylamine, dipropylamine, dibutylamine, dipentylamine and dihexylamine.

Examples of aliphatic tertiary amines include trimethylamine, triethylamine, diisopropylethylamine, tributylamine and N,N,N',N'-tetramethylethylenediamine.

Preferable examples of the "aliphatic (group)" in the above salt include methyl, ethyl and butyl, with ethyl and butyl being more preferable.

For example, the salt is preferably a salt of a tertiary amine and HF, and more preferably a salt of aliphatic tertiary amine and HF.

The salt may be, for example, a salt of the formula: R₃N•nHF or of the formula [R₃NH]⁺[F(HF)ₙ₋₁]⁻, wherein
R each independently is (cyclo)alkyl (optionally substituted with one or more halogen atoms) or
three Rs, taken together with the N to which they are attached, may form a nitrogen-containing heterocyclic ring optionally substituted with at least one (cyclo)alkyl group (optionally substituted with one or more halogen atoms); and
n is a number of 1-9 (e.g., 1, 2, 3, 4, 5, 6, 7, 8 or 9).

The salt of an amine and HF is particularly preferably a salt of trimethylamine and HF.

In another preferable embodiment of the present invention, the amine b), the fluoride c) and IF₅ d) are used in the form of a complex of the amine b), the fluoride c) and IF₅.

This complex can be used as all or part of the amine b), the fluoride c) and IF₅.

In other words, at least one member of the amine b), the fluoride c) and IF₅ may also be used, in addition to the complex.

Preferable examples of the complex include an IF₅-HF-pyridine complex.

The IF₅-HF-pyridine complex is composed of (1) IF₅, (2) 1 mol of pyridine per mol of IF₅, and (3) 1 mol of HF per mol of IF₅.

IF₅-pyridine-HF is generally produced in accordance with the method disclosed in S. Hara, M. Monoi, R. Umemura, C. Fuse, Tetrahedron, 2012, 68, 10145-10150.

Specifically, IF₅-pyridine-HF is obtained by mixing IF₅ with pyridine-HF (pyridine 50 mol%, HF 50 mol%). Pyridine-HF (pyridine 50 mol%, HF 50 mol%) is obtained by adding pyridine to an equimolar amount of anhydrous HF.

In step A the upper limit of the reaction temperature is preferably 100°C and more preferably 70°C, the lower limit is preferably -20°C and more preferably 0°C, and the reaction temperature is preferably -20°C to 100°C, and more preferably 0-70°C, and is preferably room temperature.

The reaction of step A may be stopped, as desired, by adding e.g. NaHCO₃.

In step A the upper limit of the reaction time is preferably 24 h, more preferably 12 h, and still more preferably 5 h, the lower limit is preferably 1 min., more preferably 10 min., and still more preferably 30 min., and the reaction time is preferably 1 min. to 24 hours, more preferably 10 min. to 12 h, and still more preferably 30 min. to 5 h.

The difluoromethylene compound containing a -CF₂-moiety produced in step A may be purified, as desired, by conventional purification methods, such as concentration, extraction, distillation, solvent washing, column treatment, and combinations thereof.

Step A may comprise:
step A1 of reacting the halogenated fluorine compound (a) with the sulfur chloride (b); and
step A2 of reacting the carbonyl compound of formula (2) with the reaction product of step A1.

Step A1 and step A2 can be performed sequentially or simultaneously.

In step A1 the upper limit of the reaction temperature is preferably 100°C and more preferably 70°C, the lower limit is preferably -50°C, more preferably -30°C, and still more preferably -20°C, and the reaction temperature is preferably - 20°C to 100°C, and more preferably 0-70°C, and is preferably room temperature.

Step A1 may be carried out in the presence or absence of a reaction solvent.

Specific examples of the reaction solvent include dichloromethane, tetrachloroethane, chloroform, carbon tetrachloride, cyclohexane, and mixed solvents of two or more of these.

The reaction product obtained in step A1 may be separated or purified, as desired, by using a conventional method, such as extraction, before subjecting the reaction product to step A2. Alternatively, the reaction product obtained in step A1 may be suitably used directly in step A2.

In step A1 the upper limit of the reaction time is preferably 24 h, more preferably 12 h, and still more preferably 5 h, the lower limit is preferably 1 min., more preferably 10 min., and still more preferably 30 min., and the reaction time is preferably 1 min. to 24 h, more preferably 10 min. to 12 h, and still more preferably 30 min. to 5 h.

In step A2 the upper limit of the reaction temperature is preferably 100°C and more preferably 70°C, the lower limit is preferably -20°C and more preferably 0°C, and the reaction temperature is preferably -20°C to 100°C, and more preferably 0-70°C, and is preferably room temperature.

Step A2 may be carried out in the presence or absence of a reaction solvent.

Specific examples of the reaction solvent include dichloromethane, tetrachloroethane, chloroform, carbon tetrachloride, cyclohexane, and mixed solvents of two or more of these.

In step A2 the upper limit of the reaction time is preferably 48 h, more preferably 24 h, even more preferably 10 h, and still even more preferably 5 h, the lower limit is preferably 5 min., more preferably 30 min., and still more preferably 1 h, and the reaction time is preferably 5 min. to 48 h, more preferably 30 min. to 24 h, still more preferably 1-10 h, and even more preferably 1-5 h.

In steps A, A1 and A2, an excessively low reaction temperature may cause insufficient reaction, and an excessively high reaction temperature is disadvantageous in view of costs and may cause undesirable reaction.
In steps A, A1 and A2 an excessively short reaction time may cause insufficient reaction of step A2, and an excessively long reaction time is disadvantageous in view of costs and may cause undesirable reaction.

Steps A1 and A2 above may be performed in one pot.

In this embodiment, a complex formed of the amine b), HF and IF₅ can be used.

In one embodiment of the invention, steps A1 and A2 are performed sequentially.

More specifically, the reaction of step A2 above can be initiated after the completion of the reaction of step A1 above.

In another embodiment, steps A1 and A2 are performed simultaneously.

More specifically, the reaction of step A2 above can be initiated before the reaction of step A1 above is completed.

In this embodiment, a complex formed of the amine b), HF and IF₅ can be used.

The present method can be conducted, for example, by placing a complex of the amine b), HF, and IF₅, as well as the carbonyl compound a), in a container, followed by addition of the sulfur chloride e) to the container.

For example, the present method may also be conducted by allowing the reaction of step A1 to proceed in a first container, and allowing the reaction of step A2 to proceed by placing the carbonyl compound a)in a second container, connecting the first container and the second container to allow the substances contained in these containers to be brought into contact with each other.

The difluoromethylene compound produced by the present method can be purified, as desired, by a known method, such as extraction.

The conversion percentage of the present method is preferably ≥ 50%, more preferably IF₅ 70%, and still more preferably ≥ 90%.

According to the present method, the difluoromethylene compound is produced in a yield of preferably ≥ 30%, more preferably ≥ 50%, and still more preferably ≥ 70%, and is produced with a selectivity of preferably ≥ 50%, more preferably ≥ 60%, and still more preferably ≥ 70%.

### Composition

The method of the present invention provides a difluoromethylene compound containing a -CF₂- moiety, and a composition comprising a chlorofluoromethylene compound containing a -CHI-CFCl- moiety.

The term "chlorofluoromethylene" in the chlorofluoromethylene compound refers to the -CFCl- moiety in the -CHI-CFCl- moiety.

According to the present method, a difluoromethylene compound containing a -CHX¹-CFX²- moiety can also be produced, in addition to the difluoromethylene compound.

Therefore, the composition can be produced by using the present method described above.

### Examples

The present invention is described below in more detail with reference to Examples.

### Example 1

2.0 mL (3.4 g: 25 mmol) of S₂Cl₂ was placed in a first autoclave (volume: 200 mL). After being hermetically sealed, the autoclave was cooled to -78°C, and the pressure was reduced. Thereafter, 3.4 mL (11 g, 50 mmol) of IF₅ was slowly added thereto dropwise. The autoclave was removed from a dry ice-acetone bath and left to stand at room temperature. Forty minutes later, the temperature reached about 0°C, and the pressure was increased to 0.6 MPa, and thereby gas generation was confirmed.

2.9 g (25 mmol) of ethyl pyruvate, 0.5 g (25 mmol) of hydrogen fluoride, and 5 mL of dichloromethane were placed in a second autoclave. After the autoclave was cooled to -78°C, and the internal pressure of the autoclave was reduced, this autoclave was connected to the first autoclave to allow the generated gas to be transferred. When the pressure of the first autoclave decreased to the atmospheric pressure or less, the valve was closed, and stirring was performed in the second autoclave at room temperature overnight.

The reaction solution was collected and quenched with NaHCO₃, followed by GC analysis. The conversion percentage was 100%, and CH₃CF₂CO₂Et was obtained with a selectivity of 55%.

### Example 2

6.6 g (20 mmol) of an IF₅-HF-pyridine complex was placed in an autoclave (volume: 200 mL), to which 5 mL of dichloromethane was added to prepare a solution. While stirring this solution, 2.32 g (20 mmol) of ethyl pyruvate was added thereto, followed by dropwise addition of 0.8 ml (10 mmol) of S₂Cl₂. The temperature rise or gas generation was not observed. After being hermetically sealed, the autoclave was gradually heated to 70°C in an oil bath. Twenty minutes later, the pressure was increased to 0.4 MPa, and thereby gas generation was confirmed. After heating was stopped, and the resulting product was stirred at room temperature for 1 hour, the generated gas was released, and the reaction product mixture was collected. The reaction product mixture was diluted with dichloromethane and washed twice with a potassium sulfite aqueous solution. This solution was dried over magnesium sulfate and analyzed by GC and ¹⁹F-NMR. The conversion percentage was 100%, and CH₃CF₂CO₂Et was obtained with a selectivity of 73%.

### Example 3

3.85 g (12 mmol) of an IF₅-HF-pyridine complex was placed in an autoclave (volume: 200 mL), to which 2.5 mL of dichloromethane was added to prepare a solution. While stirring, 1.06 g (10 mmol) of benzaldehyde was added to the solution, followed by dropwise addition of 0.48 mL (6 mmol) of S₂Cl₂. The temperature rise or gas generation was not observed.

After being hermetically sealed, the autoclave was gradually heated to 70°C in an oil bath. Twenty minutes later, the pressure was increased to 0.3 MPa, and thereby gas generation was confirmed.

After stirring for 3 hours, the generated gas was released, and the reaction product mixture was collected. The reaction product mixture was diluted with dichloromethane and washed twice with a potassium sulfite aqueous solution.

This solution was dried over magnesium sulfate and analyzed by GC and ¹⁹F-NMR. The conversion percentage was 54% and PhCF₂H was obtained with a selectivity of 73%.

### Example 4

3.85 g (12 mmol) of an IF₅-HF-pyridine complex was placed in an autoclave (volume: 200 mL), to which 2.5 mL of dichloromethane was added to prepare a solution. While stirring, 1.9 mL (10 mmol) of 2-decanone was added to the solution, followed by dropwise addition of 0.48 mL (6 mmol) of S₂Cl₂. The temperature rise or gas generation was not observed.

After being hermetically sealed, the autoclave was gradually heated to 70°C in an oil bath. Twenty minutes later, the pressure was increased to 0.2 MPa, and thereby gas generation was confirmed.

After stirring for 3 hours, the generated gas was released, and the reaction product mixture was collected. The reaction product mixture was diluted with dichloromethane and washed twice with a potassium sulfite aqueous solution.

This solution was dried over magnesium sulfate and analyzed by GC and ¹⁹F-NMR. The conversion percentage was 91%, and CH₃CF₂(CH₂)₆CH₃ was obtained with a selectivity of 11%.

### Example 5

3.85 g (12 mmol) of an IF₅-HF-pyridine complex was placed in an autoclave (volume: 200 mL), to which 2.5 mL of dichloromethane was added to prepare a solution. While stirring, 1.03 mL (10 mmol) of cyclohexanone was added to the solution, followed by dropwise addition of 0.48 mL (6 mmol) of S₂Cl₂. The temperature rise or gas generation was not observed.

After being hermetically sealed, the autoclave was gradually heated to 70°C in an oil bath. Twenty minutes later, the pressure was increased to 0.3 MPa, and thereby gas generation was confirmed.

After stirring at 40°C for 12 hours, the generated gas was released, and the reaction product mixture was collected. The reaction product mixture was diluted with dichloromethane and washed twice with a potassium sulfite aqueous solution.

This solution was dried over magnesium sulfate and analyzed by GC and ¹⁹F-NMR. The conversion percentage was 84%, and 1,1-difluorocyclohexane was obtained with a selectivity of 27%.

### Example 6

7.70 g (24 mmol) of an IF₅-HF-pyridine complex was placed in an autoclave (volume: 200 mL), to which 10 mL of dichloromethane was added to prepare a solution. While stirring, 1.36 g (10 mmol) of p-toluic acid was added thereto, followed by dropwise addition of 0.96 mL (12 mmol) of S₂Cl₂. The temperature rise or gas generation was not observed.

After being hermetically sealed, the autoclave was gradually heated to 70°C in an oil bath. Twenty minutes later, the pressure was increased to 0.7 MPa, and thereby gas generation was confirmed.

The temperature was gradually increased to 120°C, and stirring was performed for 12 hours. Thereafter, the generated gas was released, and the reaction product mixture was collected.

The reaction product mixture was diluted with dichloromethane and washed twice with a potassium sulfite aqueous solution.

This solution was dried over magnesium sulfate and analyzed by GC and ¹⁹F-NMR. The conversion percentage was 100%, an acid-4-methylbenzoyl fluoride was obtained with a selectivity of 81%, and 1-(trifluoromethyl)-4-methylbenzene was obtained with a selectivity of 19%.

## Claims

1. A method for producing a difluoromethylene compound containing a -CF₂- moiety, comprising step A of mixing:
a) a carbonyl compound containing a -C(O)- moiety;
b) optionally an amine;
c) a fluoride of the formula MF, wherein M is a Group 1 element of the periodic table;
d) IF₅; and
e) sulfur chloride.

2. The method of claim 1, wherein the difluoromethylene compound containing a -CF₂- moiety is a compound of the formula R¹¹-CF₂-R¹² (1), wherein
R¹¹ is R²¹ or F,
R¹² is R²² or F,
R²¹ and R²² each independently are H or an organic group, or R²¹ and R²² taken together with the -CF₂-moiety to which they are attached, may form a ring;
with the proviso that neither R¹¹ nor R¹², and neither R²¹ nor R²², is an organic group bonded via -O-; and
the carbonyl compound (a) is a compound of the formula R²¹-C(O)-R²² (2) wherein R²¹ and R²² are as defined above.

3. The method of claim 1 or 2, which comprises mixing the amine (b) and the fluoride (c).

4. The method of claim 3, wherein the amine (b) and the fluoride (c) are used in form of a salt of (b) and (c).

5. The method of any of claims 1-4, wherein the amine (b), the fluoride (c) and IF₅ (d) are used in form of a complex of (b), (c) and (d).

6. The method of any of claims 1-5, wherein the fluoride (c) is HF.

7. The method of any of claims 1-6, wherein step A comprises the steps of
A1 reacting IF₅ (d) with the sulfur chloride (e); and
A2 reacting the carbonyl compound (a) with a reaction product of step A1.

8. The method of claim 7, wherein steps A1 and A2 are performed in one pot.

9. The method of claim 8, wherein steps A1 and A2 are performed sequentially.

10. The method of claim 8, wherein steps A1 and A2 are performed simultaneously.

## Patentansprüche

1. Verfahren zur Herstellung einer Difluormethylenverbindung, die eine -CF₂-Einheit enthält, umfassend Schritt A des Mischens:
a) einer Carbonylverbindung, die eine -C(O)-Einheit enthält;
b) gegebenenfalls eines Amins;
c) eines Fluorids der Formel MF, worin M ein Element der Gruppe 1 des Periodensystems ist;
d) IF₅; und
e) Schwefelchlorid.

2. Verfahren gemäß Anspruch 1, worin die
Difluormethylenverbindung, die eine -CF₂-Einheit enthält, eine Verbindung der Formel R¹¹-CF₂-R¹² (1) ist, worin
R¹¹ für R²¹ oder F steht,
R¹² für R²² oder F steht,
R²¹ und R²² jeweils unabhängig für H oder eine organische Gruppe stehen, oder R²¹ und R²² zusammengenommen mit der -CF₂-Einheit, an die sie gebunden sind, einen Ring bilden können;
mit der Maßgabe, dass weder R¹¹ noch R¹² und weder R²¹ noch R²² eine über -O- gebundene organische Gruppe ist; und
die Carbonylverbindung (a) eine Verbindung der Formel R²¹-C(O)-R²² (2) ist, worin R²¹ und R²² wie oben definiert sind.

3. Verfahren gemäß Anspruch 1 oder 2, das das Mischen des Amins (b) und des Fluorids (c) umfasst.

4. Verfahren gemäß Anspruch 3, worin das Amin (b) und das Fluorid (c) in Form eines Salzes von (b) und (c) verwendet werden.

5. Verfahren gemäß einem der Ansprüche 1-4, worin das Amin (b), das Fluorid (c) und IF₅ (d) in Form eines Komplexes von (b), (c) und (d) verwendet werden.

6. Verfahren gemäß einem der Ansprüche 1-5, worin das Fluorid (c) HF ist.

7. Verfahren gemäß einem der Ansprüche 1-6, worin Schritt A die Schritte
A1 des Umsetzens von IF₅ (d) mit dem Schwefelchlorid (e); und
A2 des Umsetzens der Carbonylverbindung (a) mit dem Reaktionsprodukt von Schritt A1
umfasst.

8. Verfahren gemäß Anspruch 7, worin Schritte A1 und A2 in einem Kessel durchgeführt werden.

9. Verfahren gemäß Anspruch 8, worin Schritte A1 und A2 der Reihe nach durchgeführt werden.

10. Verfahren gemäß Anspruch 8, worin Schritte A1 und A2 gleichzeitig durchgeführt werden.

## Revendications

1. Procédé de production d'un composé de difluorométhylène contenant un fragment -CF₂-, comprenant une étape A consistant à mélanger :
a) un composé carbonylé contenant un fragment -C(O)- ;
b) facultativement une amine ;
c) un fluorure de formule MF, dans laquelle M est un élément du groupe 1 du tableau périodique ;
d) IF₅ ; et
e) du chlorure de soufre.

2. Procédé selon la revendication 1, dans lequel le composé de difluorométhylène contenant un fragment -CF₂- est un composé de formule R¹¹-CF₂-R¹² (1), dans laquelle
R¹¹ est R²¹ ou F,
R¹² est R²² ou F,
R²¹ et R²² sont chacun indépendamment H ou un groupe organique, ou R²¹ et R²² pris conjointement avec le fragment -CF₂- auquel ils sont liés, peuvent former un cycle ;
à condition que ni R¹¹ ni R¹², et ni R²¹ ni R²², ne soient un groupe organique lié via -O- ; et
le composé carbonylé (a) est un composé de formule R²¹-C(O)-R²² (2) dans laquelle R²¹ et R²² sont tels que précédemment définis.

3. Procédé selon la revendication 1 ou 2, qui comprend le mélange de l'amine (b) et du fluorure (c).

4. Procédé selon la revendication 3, dans lequel l'amine (b) et le fluorure (c) sont utilisés sous la forme d'un sel de (b) et (c).

5. Procédé selon l'une quelconque des revendications 1-4, dans lequel l'amine (b), le fluorure (c) et IF₅ (d) sont utilisés sous la forme d'un complexe de (b), (c) et (d).

6. Procédé selon l'une quelconque des revendications 1-5, dans lequel le fluorure (c) est HF.

7. Procédé selon l'une quelconque des revendications 1-6, dans lequel l'étape A comprend les étapes
A1 de réaction de IF₅ (d) avec le chlorure de soufre (e) ; et
A2 de réaction du composé carbonylé (a) avec un produit de réaction de l'étape A1.

8. Procédé selon la revendication 7, dans lequel les étapes A1 et A2 sont effectuées de manière monotope.

9. Procédé selon la revendication 8, dans lequel les étapes A1 et A2 sont effectuées de manière séquentielle.

10. Procédé selon la revendication 8, dans lequel les étapes A1 et A2 sont effectuées simultanément.
